(19) Europäisches Patentamt
European Patent Office
Office européen des brevets

(11) EP 2 491 925 A1

(12) EUROPEAN PATENT APPLICATION

(43) Date of publication:
29.08.2012 Bulletin 2012/35

(51) Int Cl.:
*A61K 31/337* (2006.01) *A61K 33/24* (2006.01)
*A61P 35/00* (2006.01)

(21) Application number: 11305204.7

(22) Date of filing: 25.02.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Aventis Pharma S.A.
92160 Antony (FR)

(72) Inventors:
- Bissery, Marie-Christine
75008 Paris (FR)
- Dedieu, Jean-François
75008 Paris (FR)
- Khan, Akbar
75008 Paris (FR)
- Vrignaud, Patricia
75008 Paris (FR)

(74) Representative: Gaslonde, Aude et al
Sanofi-Aventis
Département Brevets
174, Avenue de France
75013 Paris (FR)

# (54) Antitumoral combination comprising cabazitaxel and cisplatin

(57) The present invention relates to an antitumoral combination comprising cabazitaxel, which may be in the form of anhydrous base, a hydrate or a solvate, and cisplatin. The present invention relates also to a pharmaceutical composition containing such a combination and to the use of this combination and/or pharmaceutical composition in the treatment of neoplastic diseases, more particularly in the treatment of cancer.

EP 2 491 925 A1

## Description

The present invention relates to an antitumoral combination comprising cabazitaxel, which may be in the form of anhydrous base, a hydrate or a solvate, and cisplatin. The present invention relates also to a pharmaceutical composition containing such a combination and to the use of this combination and/or pharmaceutical composition in the treatment of neoplastic diseases, more particularly in the treatment of cancer.

[State of the art]

WO96/30355 discloses taxoids derivatives, among which cabazitaxel, useful as antitumoral agents. This document discloses also a long list of other drugs that may be used as a co-treatment with such taxoids. In this list, platinum complexes such as cisplatin are cited. No data supporting such co-treatment are disclosed in this document.
W02010/128258 discloses an antitumoral combination comprising cabazitaxel and capecitabine in the treatment of metastatic breast cancer for patients progressing after a previous treatment by anthracyclines and taxanes.
A phase I study is currently on going to evaluate the effects of combining cabazitaxel with cisplatin given every 3 weeks in patients with advanced solid cancer (clinicaltrials.gov site) but today no results are publicly available.

[Technical problem]

There is always a need to find new antitumoral treatments.
The invention answer to that need by providing an antitumoral combination comprising cabazitaxel, which may be in the form of anhydrous base, a hydrate or a solvate, and cisplatin. In fact, it has now been demonstrated that the efficacy of cabazitaxel may be considerably improved when it is administered in combination with cisplatin.

[Brief description of the invention]

The present invention relates to an antitumoral combination comprising cabazitaxel, which may be in the form of anhydrous base, a hydrate or a solvate, and cisplatin.
The present invention relates also to a pharmaceutical composition containing such a combination.
The present invention relates also to the use of this combination and/or pharmaceutical composition in the treatment of neoplastic diseases, more particularly in the treatment of cancer.

[Definitions]

Cabazitaxel is an antitumoral agent of the taxoid family and has the following formula:

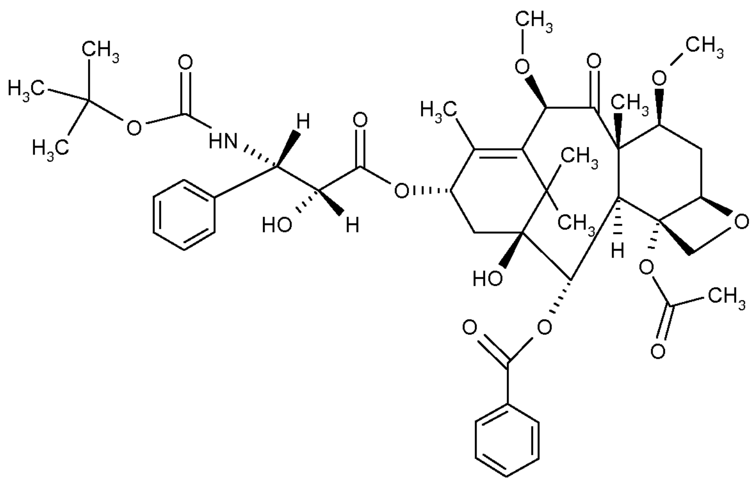

It may be in the form of anhydrous base, a hydrate or a solvate.
The chemical name of cabazitaxel is 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate. Cabazitaxel is synonymously known as (2α,5β,7β,10β,13α)-4-acetoxy-13-({(2R,3S)-3-[(tertbutoxycarbonyl)amino]-2-hydroxy-3-phenylpropanoyl}oxy)-1-hydroxy-7,10-dimethoxy-9-oxo-5,20-epoxytax-11-en-2-yl benzoate.

This compound and a preparative method thereof is described in WO96/30355, EP0817779B1 and US5847170.

Cabazitaxel may be administered in base form (cf. above formula), or in the form of a hydrate. It may also be a solvate, i.e. a molecular complex characterized by the incorporation of the crystallization solvent into the crystal of the molecule of the active principle (see in this respect page 1276 of J. Pharm. Sci. 1975, 64(8), 1269-1288).

In particular, it may be an acetone solvate, and, more particularly, may be the solvate described in W02005/02846. It may be an acetone solvate of cabazitaxel containing between 5% and 8% and preferably between 5% and 7% by weight of acetone (% means content of acetone/content of acetone+cabazitaxel × 100). An average value of the acetone content is 7%, which approximately represents the acetone stoichiometry, which is 6.5% for a solvate containing one molecule of acetone. The procedure described below allows the preparation of an acetone solvate of cabazitaxel: 940 ml of purified water are added at 20±5°C (room temperature) to a solution of 207 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate at about 92% by weight in about 2 litres of acetone, followed by seeding with a suspension of 2 g of 4α-acetoxy-2α-benzoyloxy-5β,20-epoxy-1β-hydroxy-7β,10β-d i methoxy-9-oxo-11-taxen-13a-yl (2 R, 3S)-3-*tert*-butoxycarbonylamino-2-hydroxy-3-phenylpropionate isolated from acetone/water in a mixture of 20 ml of water and 20 ml of acetone. The resulting mixture is stirred for about 10 to 22 hours, and 1.5 litres of purified water are added over 4 to 5 hours. This mixture is stirred for 60 to 90 minutes, and the suspension is then filtered under reduced pressure. The cake is washed on the filter with a solution prepared from 450 ml of acetone and 550 ml of purified water, and then oven-dried at 55°C under reduced pressure (0.7 kPa) for 4 hours. 197 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate acetone containing 0.1% water and 7.2% acetone (theoretical amount: 6.5% for a stoichiometric solvate) are obtained.

Cabazitaxel may be administered parenterally, such as via intravenous administration. A galenical form of cabazitaxel suitable for administration by intravenous infusion is that in which the cabazitaxel is dissolved in water in the presence of excipients chosen from surfactants, cosolvents, glucose or sodium chloride, etc. For example, a galenical form of cabazitaxel may be prepared by diluting a premix solution of cabazitaxel contained in a sterile vial (80 mg of cabazitaxel + 2 ml of solvent + Polysorbate 80) with a sterile vial containing a solution of 6 ml of water and ethanol (13% by weight of 95% ethanol) in order to obtain 8 ml of a solution ready to be rediluted in a perfusion bag. The concentration of cabazitaxel in this ready-to-redilute solution is about 10 mg/ml. The perfusion is then prepared by injecting the appropriate amount of this ready-to-redilute solution into the perfusion bag containing water and glucose (about 5%) or sodium chloride (about 0.9%).

Cisplatin is a platinum derivative used to treat various types of cancers, including sarcomas, some carcinomas (e.g. small cell lung cancer, and ovarian cancer), lymphomas, and germ cell tumors. It was the first member of a class of anti-cancer drugs which now also includes carboplatin and oxaliplatin. These platinum complexes react in vivo, binding to and causing crosslinking of DNA which ultimately triggers apoptosis (programmed cell death).

Effective quantity: quantity of a pharmaceutical compound producing an effect on the treated tumor.

Therapeutic synergy:

The improved efficacy of the combination according to the invention may be demonstrated by the determination of the therapeutic synergy.

A combination manifests therapeutic synergy if it is therapeutically superior to the best agent of the study used alone at its maximum tolerated dose (HNTD or Highest Non Toxic Dose) or at its highest dose tested when toxicity cannot be reached in the animal species.

This efficacy may be quantified, for example, by the $\log_{10}$ cell kill, which is determined according to the following formula:

$$\log_{10} \text{ cell kill} = \text{T-C (days)}/3.32 \times T_d$$

in which T - C represents the tumor growth delay, which is the median time in days for the tumors of the treated group (T) and the tumors of the control group (C) to have reached a predetermined value (1 g for example), and $T_d$ represents the time in days needed for the volume of the tumor to double in the control animals [T.H. Corbett et al., Cancer, 40: 2660-2680 (1977); F.M. Schabel et al., Cancer Drug Development, Part B, Methods in Cancer Research, 17: 3-51, New York, Academic Press Inc. (1979)].

A product is considered to be active if $\log_{10}$ cell kill is greater than or equal to 0.7. A product is considered to be very active if $\log_{10}$ cell kill is greater than or equal to 2.8.

The combination will manifest therapeutic synergy when the $\log_{10}$ cell kill is greater than the value of the $\log_{10}$ cell kill of the best constituent administered alone at its maximum tolerated dose (by at least 1 log cell kill).

The efficacy of the combinations on solid tumors may be determined experimentally in the following manner:

The animals subjected to the experiment, generally mice, are subcutaneously grafted bilaterally with 30 to 60 mg of a tumor fragment on day 0. The animals are implanted with a murine tumor grafted in the syngenic strain of mice of origin of the tumor, or by a rodent or human tumor xenografted in immunocompromized mice. Some days post tumor implantation, mice are randomized according to their body weight to the different groups of treatments and controls. The animals are observed every day. The different animal groups are weighed daily during treatment until the maximum weight loss is reached and subsequent full weight recovery has occurred. The groups are then weighed once or twice a week until the end of the trial.

The tumors are measured 1 to 5 times a week, depending on the tumor doubling time, until the tumor reaches approximately 2 g, or until the animal dies (if this occurs before the tumor reaches 2 g). The animals are necropsied immediately after euthanasia or death.

The antitumor activity is determined in accordance with the different parameters recorded.

[Description of the invention]

The present invention relates to an antitumoral combination comprising cabazitaxel, which may be in the form of anhydrous base, a hydrate or a solvate, and cisplatin.

For example, cabazitaxel may be in the form of an acetone solvate, and, more particularly, may be the solvate described in W02005/02846.

The acetone solvate of cabazitaxel may contain between 5% and 8% and preferably between 5% and 7% by weight of acetone (% means content of acetone/content of acetone+cabazitaxel $\times$ 100). An average value of the acetone content is 7%, which approximately represents the acetone stoichiometry, which is 6.5% for a solvate containing one molecule of acetone.

The present invention also relates to an antitumoral combination comprising an effective quantity of cabazitaxel and an effective quantity of cisplatin.

The present invention also relates to an antitumoral combination which shows therapeutic synergy.

Cabazitaxel and cisplatin may be administered simultaneously, semi-simultaneously, separately, or spaced out over a period of time so as to obtain the maximum efficacy of the combination; it may be possible for each administration to vary in its duration from a rapid administration to a continuous perfusion.

As a result, for the purposes of the present invention, the combination is not exclusively limited to the one which is obtained by physical association of the constituents, but also to those which permit a separate administration, which can be simultaneous or spaced out over a period of time.

In the combinations according to the invention, the application of the constituents of which may be simultaneous, separate or spaced out over a period of time, it is especially advantageous for the amount of cabazitaxel to represent from 10 to 90% by weight of the combination, it being possible for this content to vary in accordance with the nature of the associated substance, the efficacy sought and the nature of the cancer to be treated.

In the combination according to the invention, cabazitaxel and cisplatin are preferably administered parentally.

The use of cabazitaxel and cisplatin for the preparation of a combination according to the invention is also part of the invention.

The present invention also relates to a pharmaceutical composition containing the combination according to the invention.

The present invention relates also to the combination according to the present invention for its use as a medicament in the treatment of neoplastic diseases, more particularly in the treatment of cancer.

The present invention relates also to the pharmaceutical composition according to the present invention for its use as a medicament in the treatment of neoplastic diseases, more particularly in the treatment of cancer.

The improved efficacy of the combination according to the invention may be demonstrated by determination of the therapeutic synergy as illustrated in the following example.

Example:

In this example, the effectiveness of a cabazitaxel/cisplatin combination of the invention for tumor growth inhibition was demonstrated in vivo.

The selected tumor model was a murine tumor, colon adenocarcinoma C51, grafted in the syngenic strain of mice of origin of the tumor, BALB/C mice [T.H. Corbett et al., Cancer, 40: 2660-2680 (1977)].

Cabazitaxel was weighed for each treatment and dissolved in ethanol. Treatment solutions were prepared first by mixing 1 volume of ethanolic stock solution and 1 volume of polysorbate 80, then by adding 18 volumes of glucose 5% in water.

Cabazitaxel was administered intravenously on days 5, 12 (or 13) after tumor implantation. Cisplatin was

formulated in NaCl 0.9%, pH 4.5. Cisplatin was administered intravenously on days 5, 12 (or 13) after tumor implantation, with a 15 min or a 24h interval to cabazitaxel.

The results of the experiment are reported in Table 1.

Tumor doubling time was 2.5 days.

The following end points have been used:

- Toxicity was declared at dosages inducing ≥ 20% body weight loss or ≥ 10 % drug death Tumor growth inhibition was determined on day 20 post tumor implantation when the median tumor size in the control group was 1352 mg.

The tumors of treatment (T) and control (C) groups were measured when the median of control group reached approximately 750 to 1400 mg. The median tumor weight of each group was determined.

- Antitumor efficacy was determined by calculating the T/C value in percent:

$$T/C\ (\%) = \text{Median tumor weight of the Treated/ control} \times 100$$

According to NCI standards, a T/C < 42 % is the minimal level to declare activity. A T/C < 10 % is considered to indicate high antitumor activity and is the level used by NCI to justify further development (Decision Network-2 level, DN-2).

- To better quantify the antitumor activity, another end point, the log cell kill, was used.:

$$\log10 \text{ cell kill} = (T-C) / [3.32 \times (\text{tumor doubling time in days})]$$

  (T meaning the median time of the treated mice to reach 750 mg and C the median time (17.6 days) of the control mice to reach the same size). No antitumor activity was declared for log cell kill < 0.7, and the treatment was declared highly active for log cell kill ≥ 2.8
- Therapeutic Synergism: a combination has therapeutic synergism if it is more active than the best single agent of the study (by at least 1 log cell kill).

Toxicity for cabazitaxel alone was observed at a dose of 32.3 mg/kg/injection, with 6/6 drug-related deaths occurring from day 19 to day 24. The highest nontoxic dose (HNTD) for cabazitaxel, 20 mg/kg/inj (total injected dose = 40 mg/kg), was found to be active with a log cell kill of 1.8. The lowest doses of 12.4 and 7.7 mg/kg/inj remained active with 1.4 and 1.3 log cell kill, respectively.

Toxicity for cisplatin alone was observed at a dose of 7 mg/kg/injection, with 2/6 drug-related deaths occurring on days 8 and 23, a 24.6% body weight loss being also observed at nadir on day 17, i.e. above the 20% threshold. The HNTD for cisplatin, 4.3 mg/kg/inj (total injected dose = 8.6 mg/kg), was found to be active with a log cell kill of 2.7. The lowest dose 2.7 mg/kg/inj remained active with 1.8 log cell kill.

Using a 15 min interval between administrations of the 2 agents, the combination of cisplatin at 3.5 mg/kg/inj with cabazitaxel at 10 mg/kg/inj was declared toxic, with 19.5 % body weight loss at nadir on day 20. The dose of 2.8 mg/kg/inj of cisplatin combined with 8 mg/kg/inj of cabazitaxel was considered to be the HNTD. Remarkably, this dose was found highly active with 4.6 log cell kill, clearly far more active than the activity of each agent administered alone. Likewise, a greater antitumor activity was also observed at the dose below the HNTD (2.1 mg/kg/inj of cisplatin with 6 mg/kg/inj of cabazitaxel) with 3.6 log cell kill.

We can thus conclude that this combination shows a therapeutic synergy.

Using a 24 h interval between administrations of the 2 agents, two different order of administrations have been evaluated, cabazitaxel first on days 5 and 12, cisplatin being administered 24h later on days 6 and 13, or the reverse sequence with cisplatin being administered first.

- Cabazitaxel first: The combination of cabazitaxel at 13 mg/kg/inj with cisplatin at 4.6 mg/kg/inj was declared toxic, with 1/6 drug-related death, and 27.5 % body weight loss at nadir on day 21. The dose of cabazitaxel at 10 mg/kg/inj followed 24h later by cisplatin at 3.5 mg/kg/inj was considered to be the HNTD. This dose was found highly active with 4.6 log cell kill, being clearly far more active than the activity of each agent administered alone, as previously observed in the 15min interval combination. Of interest, a greater antitumor activity was also observed at the 2 doses below the HNTD (cabazitaxel/cisplatin combination at 8/2.8 and 6/2.1 mg/kg/inj), with 4.1 and 3.7 log cell kill, respectively.

We can thus conclude that using this sequence of administration of 24h interval with cabazitaxel being injected first, this combination shows also a therapeutic synergy.

- Cisplatin first: The combination of cisplatin at 4.6 mg/kg/inj with cabazitaxel at 13 mg/kg/inj was declared toxic, with 20.6 % body weight loss at nadir on day 19. The dose of cisplatin at 3.5 mg/kg/inj followed 24h later by cabazitaxel at 10 mg/kg/inj was considered to be the HNTD. As previously observed when cabazitaxel was administered first, this HNTD was found highly active with 4.4 log cell kill. A greater antitumor activity was also observed at the 2 doses below the HNTD (cisplatin/cabazitaxel combination at 2.8/8 and 2.1/6 mg/kg/inj), with 4.1 and 3.5 log cell kill, respectively.

We can thus conclude that using this sequence of administration of 24h interval with cisplatin being injected first, this combination shows also a therapeutic synergy.

In conclusion, cabazitaxel-cisplatin combination shows therapeutic synergy whatever the sequence of administration of the agents (at the HNTD: 4.4 to 4.6 log cell kill for the combination versus 1.8 log cell kill for cabazitaxel alone and 2.7 log cell kill for cisplatin alone). In addition, this therapeutic synergy was maintained at the dosages below the HNTD at one (15min apart) and 2 (24h apart) dose levels.

**Table I: Combination of cabazitaxel and cisplatin against colon adenocarcinoma C51 implanted in BALB/c female mice.**

| Agent by IV route Dose in mg/kg/inj (total dose) | | Schedule in days | Drug death | % Body weight change at nadir (day) | T/C in % on day 20 | T-C in days (750 mg) | $log_{10}$ cell kill gross | Comments |
|---|---|---|---|---|---|---|---|---|
| **Cabazitaxel** | **Cisplatin** | | | | | | | |
| 32.3 (64.6) | - | 5, 12 | 6/6 | - | - | - | - | Toxic (6/6 DD) |
| 20.0 (40.0) | - | | 0/6 | -14.6 (19) | 0 | 15.2 | 1.8 | HNTD active |
| 12.4 (24.8) | - | | 0/6 | -8.1 (9) | 3 | 11.7 | 1.4 | Active |
| 7.7 (15.4) | - | | 0/6 | -6.8 (9) | 5 | 11.0 | 1.3 | Active |
| - | 7.0 (14.0) | 5,12 | 2/6 | -24.6 (17) | - | - | - | Toxic (2/6 DD; -25% BWC) |
| - | 4.3 (8.6) | | 0/6 | -7.8 (15) | 0 | 22.1 | 2.7 | HNTD active |
| - | 2.7 (5.4) | | 0/6 | -3.7 (7) | 2 | 15.1 | 1.8 | Active |
| **15 min interval com bination** | | | | | | | | |
| 10.0 (20.0) | 3.5 (7.0) | 5, 12 | 0/6 | -19.5 (20) | - | - | - | Toxic (-20% BWC) |
| 8.0 (16.0) | 2.8 (5.6) | | 0/6 | -9.3(18) | 0 | 38.4 | 4.6 | HNTD highly active |
| 6.0 (12.0) | 2.1 (4.2) | | 0/6 | -7.1 (9) | 0 | 30.1 | 3.6 | Highly active |
| 3.0 (6.0) | 1.1 (2.2) | | 0/6 | -3.4 (7) | 4 | 12.2 | 1.5 | Active |
| **24h interval combi nation, cabazitaxel, first** | | | | | | | | |
| 13.0 (26.0) | 4.6 (9.2) | 5, 12 | 1/6 | -27.5 (21) | - | - | - | Toxic (-28% BWC) |
| 10.0 (20.0) | 3.5 (7.0) | | 0/6 | -17.9 (17) | 0 | 37.8 | 4.6 | HNTD highly active |

(continued)

| | | | | | | | | |
|---|---|---|---|---|---|---|---|---|
| **24h interval combi nation, cabazitaxel, first** | | | | | | | | |
| 8.0 (16.0) | 2.8 (5.6) | | 0/6 | -17.2 (21) | 0 | 34.1 | 4.1 | Highly active |
| 6.0 (12.0) | 2.1 (4.2) | | 0/6 | -11.1 (19) | 0 | 30.3 | 3.7 | Highly active |
| 3.0 (6.0) | 1.1 (2.2) | | 0/6 | -4.3 (15) | 4 | 11.3 | 1.4 | Active |
| **24h interval combi nation, cisplatin, first** | | | | | | | | |
| 13.0 (26.0) | 4.6 (9.2) | 5, 12 | 0/6 | -20.6 (19) | - | - | - | Toxic (-21% BWC) |
| 10.0 (20.0) | 3.5 (7.0) | | 0/6 | -14.4 (17) | 0 | 36.2 | 4.4 | HNTD highly active |
| 8.0 (16.0) | 2.8 (5.6) | | 0/6 | -16.3 (19) | 0 | 34.3 | 4.1 | Highly active |
| 6.0 (12.0) | 2.1 (4.2) | | 0/6 | -7.0 (18) | 0 | 29.3 | 3.5 | Highly active |
| 3.0 (6.0) | 1.1 (2.2) | | 0/6 | -0.9 (7) | 6 | 10.1 | 1.2 | Active |
| Tumor doubling time = 2.5 days. Median tumor size in the control group on day 20 = 1352 mg. Time for median control tumor to reach 750 mg = 17.6 days. Abbreviations used: BWC = body weight change, T/C = tumor growth inhibition, T-C = tumor growth delay, HNTD = highest nontoxic dose, IV = intravenous. | | | | | | | | |

## Claims

1. Combination comprising cabazitaxel, which may be in the form of anhydrous base, a hydrate or a solvate, and cisplatin.

2. Combination according to claim 1 were cabazitaxel is in the form of a solvate.

3. Combination according to claim 2 were the solvate is an acetone solvate.

4. Combination according to claim 3 were the acetonate solvate contains between 5% and 8% by weight of acetone.

5. Combination according to any one of claims 1 to 4 comprising an effective quantity of cabazitaxel and an effective quantity of cisplatin.

6. Combination according to any one of claims 1 to 5 where the antitumoral combination shows therapeutic synergy.

7. Combination according to any one of claims 1 to 6 where cabazitaxel and cisplatin are administered simultaneously, semi-simultaneously, separately, or spaced out over a period of time.

8. Combination according to any one of claims 1 to 7 where the amount of cabazitaxel represents from 10 to 90% by weight of the combination.

9. Combination according to any one of claims 1 to 8 where cabazitaxel and cisplatin are both administered parentally.

10. Combination according to any one of claims 1 to 9 for its use as a medicament in the treatment of cancer.

11. Use of cabazitaxel and cisplatin for the preparation of a combination according to any one of claims 1 to 9.

12. A pharmaceutical composition containing the combination according to any one of claims 1 to 9.

13. A pharmaceutical composition according to claim 12 for its use as a medicament in the treatment of cancer.

Europäisches Patentamt
European Patent Office
Office européen des brevets

# EUROPEAN SEARCH REPORT

Application Number
EP 11 30 5204

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | Anonymous: "A Study to Evaluate the Effects of Combining Cabazitaxel With Cisplatin Given Every 3 Weeks in Patients With Advanced Solid Cancer (NCT00925743)", Clinicaltrials.gov , 28 May 2010 (2010-05-28), pages 1-2, XP002639748, Retrieved from the Internet: URL:http://clinicaltrials.gov/archive/NCT00925743/2010_05_28 [retrieved on 2011-05-31] | 1-13 | INV. A61K31/337 A61K33/24 A61P35/00 |
| Y | * the whole document * | 1-13 | |
| Y,D | WO 2010/128258 A1 (SANOFI AVENTIS [FR]; MAGHERINI EMMANUELLE [FR]) 11 November 2010 (2010-11-11) * page 2, line 20 - page 3, line 8 * | 2-4 | |
| Y | AGARWAL N ET AL: "Cabazitaxel for the treatment of castration-resistant prostate cancer", FUTURE ONCOLOGY, vol. 7, no. 1, January 2011 (2011-01), pages 15-24, XP9144697, FUTURE MEDICINE LTD. GBR ISSN: 1479-6694, DOI: 10.2217/FON.10.168 * the whole document * * page 22, column 1, last 5 lines - column 2, line 2 * | 1-13 | TECHNICAL FIELDS SEARCHED (IPC): A61K A61P |

-/--

The present search report has been drawn up for all claims

| Place of search | Date of completion of the search | Examiner |
|---|---|---|
| Munich | 31 May 2011 | Rodríguez-Palmero, M |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 30 5204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CROWN JOHN ET AL: "The taxanes: An update", LANCET (NORTH AMERICAN EDITION), vol. 355, no. 9210, 1 April 2000 (2000-04-01), pages 1176-1178, XP002639750, ISSN: 0099-5355 * page 1176, column 2, paragraph 5 - page 1177, column 2 * ----- | 1-13 | |
| Y | CROWN JOHN ET AL: "Platinum-taxane combinations in metastatic breast cancer: An evolving role in the era of molecularly targeted therapy.", BREAST CANCER RESEARCH AND TREATMENT, vol. 79, no. Supplement 1, 2003, pages S11-S18, XP002639751, ISSN: 0167-6806 * abstract * ----- | 1-13 | |
| Y | MCGUIRE WILLIAM P 3RD: "Current status of taxane and platinum-based chemotherapy in ovarian cancer.", JOURNAL OF CLINICAL ONCOLOGY : OFFICIAL JOURNAL OF THE AMERICAN SOCIETY OF CLINICAL ONCOLOGY, vol. 21, no. 10 Suppl, 15 May 2003 (2003-05-15), pages 133S-135S, XP002639752, ISSN: 1527-7755 * abstract * ----- -/-- | 1-13 | TECHNICAL FIELDS SEARCHED (IPC) |

The present search report has been drawn up for all claims

| Place of search | Date of completion of the search | Examiner |
|---|---|---|
| Munich | 31 May 2011 | Rodríguez-Palmero, M |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 30 5204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RIGAS JAMES R: "Taxane-platinum combinations in advanced non-small cell lung cancer: a review.", THE ONCOLOGIST, vol. 9 Suppl 2, 2004, pages 16-23, XP002639753, ISSN: 1083-7159 * abstract * ----- | 1-13 | |
| A | BOUCHET B P ET AL: "Cabazitaxel, a new taxane with favorable properties.", DRUGS OF TODAY (BARCELONA, SPAIN : 1998), vol. 46, no. 10, October 2010 (2010-10), pages 735-742, XP002639754, ISSN: 1699-3993 * the whole document * ----- | 1-13 | |
| | | | TECHNICAL FIELDS SEARCHED (IPC) |

The present search report has been drawn up for all claims

| Place of search | Date of completion of the search | Examiner |
|---|---|---|
| Munich | 31 May 2011 | Rodríguez-Palmero, M |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 11 30 5204

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2011

| Patent document cited in search report | Publication date | Patent family member(s) | Publication date |
|---|---|---|---|
| WO 2010128258 A1 | 11-11-2010 | FR 2945211 A1 | 12-11-2010 |
| | | FR 2945212 A1 | 12-11-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9630355 A
- WO 2010128258 A
- EP 0817779 B1
- US 5847170 A
- WO 200502846 A


### Non-patent literature cited in the description


- *J. Pharm. Sci.,* 1975, vol. 64 (8), 1269-1288
- **T.H. CORBETT et al.** *Cancer,* 1977, vol. 40, 2660-2680
- **F.M. SCHABEL et al.** Cancer Drug Development, Part B, Methods in Cancer Research. Academic Press Inc, 1979, vol. 17, 3-51